# EUROPEAN PATENT APPLICATION

(11) **EP 3 195 788 A1**
(43) Date of publication of application: **26.07.2017**
(21) Application number: 16799719.6
(22) Date of filing: 20.04.2016
(51) Int. Cl.: A61B 1/00, G02B 23/24

(54) **ENDOSCOPE CURVED PORTION**

(30) Priority: 28.05.2015 JP 2015109049
(71) Applicant: Olympus Corporation, Hachioji-shi Tokyo 192-8507 (JP)
(72) Inventor: SETO, Hideyuki, Hachioji-shi Tokyo 192-8507 (JP); NARA, Takashi, Hachioji-shi Tokyo 192-8507 (JP)
(74) Representative: Schicker, Silvia
(86) International application number: PCT/JP2016/062546
(87) International publication number: WO 2016/190011

(57) **Abstract**

An endoscope bending portion includes a circular cylindrical member 20, a protrusion portion 60 that is formed protruding on an inner circumference of the circular cylindrical member 20, on a distal end side, and on which a wire hole 60h is formed, a pulling wire 40u that has a maximum outer diameter portion 41u formed at a distal end portion, and that is inserted through the wire hole 60h to have the maximum outer diameter portion 41u engaged with at least a part of a distal end surface 60s of the protrusion portion 60, and a restriction portion 60a that restricts inward movement, in a radial direction K, of the maximum outer diameter portion 41u engaged with the protrusion portion 60.

## Description

### Technical Field

The present invention relates to an endoscope bending portion including a tubular member for bending.

### Background Art

In recent years, endoscopes to be inserted into a subject have become widely used in a medical field. An endoscope used in the medical field enables observation of an organ in a body cavity of a subject by insertion of an elongated insertion section into the body cavity, or enables various treatments to be performed by using, as necessary, a treatment instrument inserted in a treatment instrument insertion channel provided to the endoscope, for example.

Furthermore, endoscopes are also used in an industrial field without being limited to the medical field. An endoscope used in the industrial field enables observation of a scratch, corrosion or the like of a part to be examined inside an object or examinations including various treatments, by insertion of an elongated insertion section of the endoscope into a jet engine or an object such as a pipe in a factory, for example.

A configuration according to which a bending portion which is bendable in a plurality of directions is provided to the insertion section of an endoscope is known, for example. The bending portion enhances the advanceability of the insertion section at a bent portion of a pipe.

Moreover, the bending portion allows the observation direction of an observation optical system, which is provided to a distal end portion on the distal end side of the bending portion in a longitudinal direction of the insertion section (hereinafter simply referred to "distal end side"), to be changed at the insertion section.

Normally, the bending portion provided to the insertion section of the endoscope is configured to be bendable in, for example, two directions of up and down or four directions of up, down, left and right by coupling, along the longitudinal direction, a plurality of bending pieces which form a tubular member for bending which is made of metal, such as stainless steel.

More specifically, the bending portion is bendable in any of up and down or up, down, left and right directions by an operation section pulling any of two or four pulling wires which are made of stainless steel or the like, which are inserted through the insertion section, and the distal ends of which in the longitudinal direction (hereinafter simply referred to as "distal end(s)") are fixed by welding, such as soldering or brazing, to a bending piece on the most distal end side among the plurality of bending pieces.

Furthermore, to reduce the diameter of the insertion section and enhance the layout of internal components of the bending portion, and also, to improve the bent shape of the bending portion, a circular cylindrical member made of a super-elastic alloy, such as a nickel-titanium alloy, is known to be used as the tubular member for bending, instead of a plurality of bending pieces.

More specifically, a configuration is known according to which the bending portion is bendable according to a pulling operation of a pulling wire, by having a plurality of slots for bending formed, at predetermined intervals along the longitudinal direction, on an outer circumference of a circular cylindrical member, on each of an UP side and a DOWN side of the bending direction of the bending portion, that is, the UP side and the DOWN side of an endoscopic image (hereinafter simply referred to as "UP side" and "DOWN side").

Note that, also with the circular cylindrical member made of a super-elastic alloy, the bending portion is preferably made bendable by connecting a distal end of a pulling wire inserted through the insertion section to a distal end on the inner circumferential surface of the circular cylindrical member and by pulling the pulling wire by the operation section.

However, if the pulling wire is made of stainless steel and the circular cylindrical member is made of a super-elastic alloy, the materials of the pulling wire and the circular cylindrical member are different. Accordingly, a problem that the distal end of the pulling wire cannot be welded to the circular cylindrical member is possibly caused.

Note that such a problem also arises when the tubular member for bending is formed from a plurality of bending pieces, and the bending piece to which the distal end of the pulling wire is to be fixed is made of a material different from the material of the wire.

With respect to such a problem, a configuration is conceivable according to which a coupling member of stainless steel, for example, is provided to the insertion section of the endoscope, between the tubular member for bending and a distal end rigid member forming a distal end portion of the insertion section in the longitudinal direction, and the distal end of the pulling wire is welded to the coupling member. However, with such a configuration, a distal end rigid length of the insertion section is increased in the longitudinal direction due to the use of the coupling member.

Accordingly, in view of such a circumstance, Japanese Patent Application Laid-Open Publication No. 2000-70217 discloses a configuration of an endoscope bending portion, a distal end rigid length of which is not increased and which allows a distal end of a pulling wire to be fixed, where a wire engaging pin through which the pulling wire is inserted along the longitudinal direction is provided on an inner circumferential surface of a bending piece on the most distal end side, among a plurality of bending pieces, and a distal end stopper, which is a maximum outer diameter portion provided to a distal end portion of the pulling wire and having a diameter larger than the diameter of the pulling wire, is engaged with a distal end surface of the wire engaging pin.

However, with the configuration of the endoscope bending portion disclosed in Japanese Patent Application Laid-Open Publication No. 2000-70217, because the distal end stopper is simply engaged with the distal end surface of the wire engaging pin, the wire engaging pin may move inward in the radial direction of the tubular member for bending when a rearward pulling force in the longitudinal direction is applied to the pulling wire to bend the bending portion. A problem that the wire engaging pin interferes with an internal component of the tubular member for bending is caused as a result.

The present invention has been made in view of the problems described above, and has its object to provide an endoscope bending portion having a configuration which allows a distal end of a pulling wire to be fixed to a tubular member for bending without affecting a distal end rigid length, and which is capable of preventing movement of a maximum outer diameter portion of the pulling wire in a radially inward direction.

### Disclosure of Invention

### Means for Solving the Problem

An endoscope bending portion according to an aspect of the present invention includes a tubular member for bending, a protrusion portion that is formed protruding on an inner circumference of the tubular member for bending, on a distal end side in a longitudinal direction, and that has a wire hole formed in the longitudinal direction, a pulling wire that has a maximum outer diameter portion formed at a distal end portion in the longitudinal direction, and that is inserted through the wire hole to have the maximum outer diameter portion engaged with at least a part of a distal end surface of the protrusion portion, and a restriction portion that is provided inside the tubular member for bending, and that restricts inward movement in the tubular member for bending, in a radial direction, of the maximum outer diameter portion engaged with the protrusion portion.

### Brief Description of the Drawings

Fig. 1 is a perspective view showing an endoscope including an endoscope bending portion of a first embodiment at an insertion section;
Fig. 2 is a perspective view showing a tubular member for bending constituting the endoscope bending portion in Fig. 1;
Fig. 3 is a diagram focusing on a circular cylindrical member, and schematically showing a part of a cross-section of the insertion section, taken along line III-III in Fig. 1;
Fig. 4 is a partial cross-sectional view of a distal end side of the insertion section, showing a modification where a protrusion portion in Fig. 3 is formed only from a guide member;
Fig. 5 is a partial cross-sectional view showing a circular cylindrical member constituting a bending portion of a second embodiment, together with a distal end rigid portion and a pulling wire, a maximum outer diameter portion of which is fixed to a distal end portion;
Fig. 6 is a partial cross-sectional view showing a circular cylindrical member constituting a bending portion of a third embodiment, together with a pulling wire, a maximum outer diameter portion of which is fixed to a distal end portion;
Fig. 7 is a partial cross-sectional view showing a circular cylindrical member constituting a bending portion of a fourth embodiment, together with a pulling wire, a maximum outer diameter portion of which is fixed to a distal end portion;
Fig. 8 is a cross-sectional view of the circular cylindrical member and a protrusion portion, taken along line VIII-VIII in Fig. 7;
Fig. 9 is a partial cross-sectional view showing a modification of the configuration of the circular cylindrical member in Fig. 8, together with a pulling wire, a maximum outer diameter portion of which is fixed to a distal end portion;
Fig. 10 is a cross-sectional view of the circular cylindrical member and a protrusion portion, taken along line X-X in Fig. 9;
Fig. 11 is a plan view schematically showing, from an UP side, an external appearance of a distal end side of a circular cylindrical member constituting a bending portion of a fifth embodiment;
Fig. 12 is a diagram showing a cross-section of the circular cylindrical member, taken along line XII-XII in Fig. 11, together with a pulling wire, a maximum outer diameter portion of which is fixed to a distal end portion;
Fig. 13 is a cross-sectional view of the circular cylindrical member and a protrusion portion, taken along line XIII-XIII in Fig. 12;
Fig. 14 is a plan view schematically showing, from an UP side, an external appearance of a distal end side of a circular cylindrical member constituting a bending portion of a sixth embodiment;
Fig. 15 is a diagram showing a cross-section of the circular cylindrical member, taken along line XV-XV in Fig. 14, together with a pulling wire, a maximum outer diameter portion of which is fixed to a distal end portion; and
Fig. 16 is a plan view schematically showing, from an UP side, an external appearance of a distal end side of a circular cylindrical member of a modification according to which a groove width in Fig. 15 is made smaller than a maximum outer diameter portion.

### Best Mode for Carrying Out the Invention

Hereinafter, embodiments of the present invention will be described with reference to the drawings. It should be noted that the diagrams are schematic and the relationship between a thickness and a width of each member, the ratio of thicknesses of members and the like are not actual, and it is needless to say that the relationship between dimensions and the ratios may be different between the drawings.

### (First Embodiment)

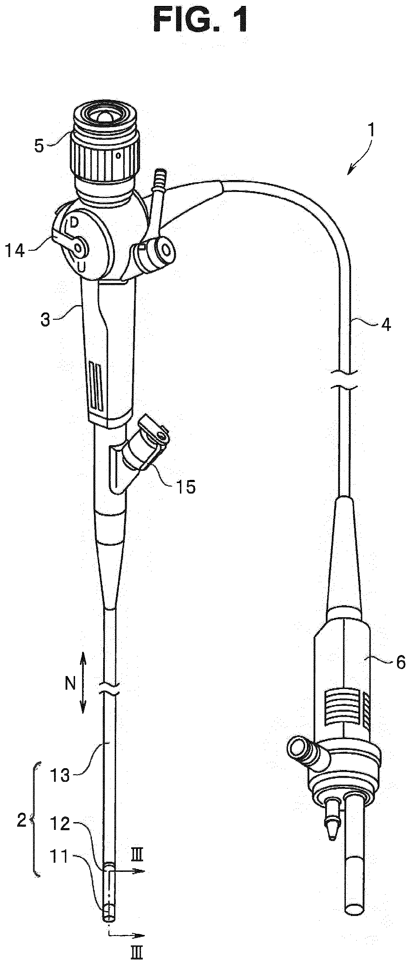

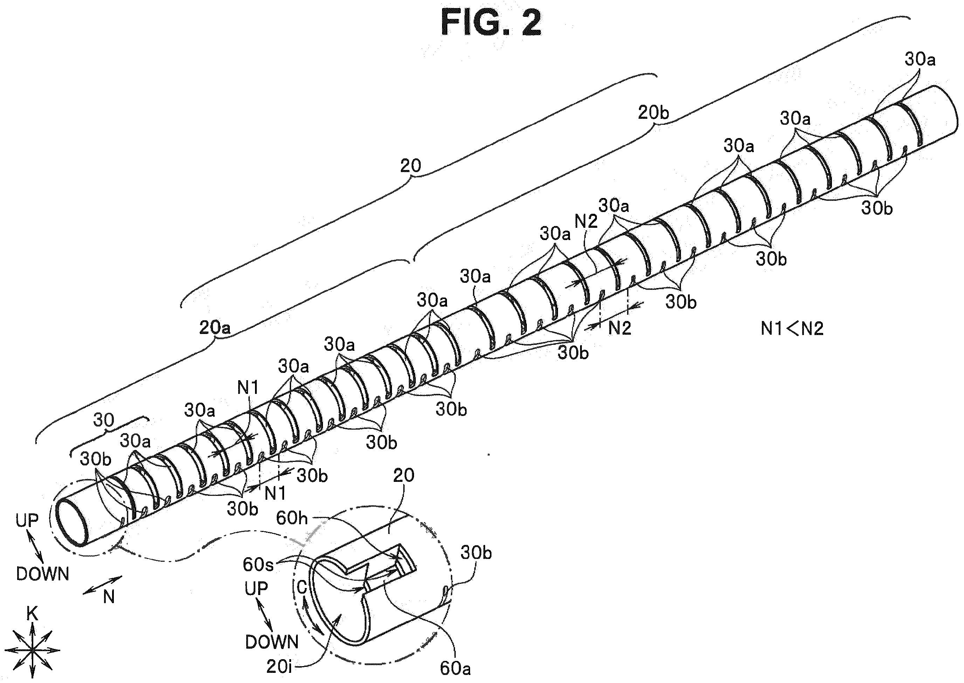

Fig. 1 is a perspective view showing an endoscope including an endoscope bending portion of a present embodiment at an insertion section, and Fig. 2 is a perspective view showing a tubular member for bending constituting the endoscope bending portion in Fig. 1.

As shown in Fig. 1, an endoscope 1 includes an insertion section 2 which is to be inserted into a subject and which is elongated along a longitudinal direction N, and an operation section 3 which is provided at a proximal end of the insertion section 2 in the longitudinal direction N (hereinafter such a proximal end will be referred to simply as "proximal end").

The endoscope 1 also includes a universal cord 4 extending from a side portion of the operation section 3, an eyepiece section 5 provided at a proximal end of the operation section 3, and a connector 6 provided at an extending end of the universal cord 4.

Note that the endoscope 1 is connectable to an external device, such as a light source device, due to the connector 6 being attachable/detachable to/from the external device.

Main components of the insertion section 2 include a distal end portion 11 at a distal end side, an endoscope bending portion (hereinafter simply referred to as "bending portion") 12 which is continuously provided to a proximal end of the distal end portion 11, and a flexible tube portion 13, having flexibility, which is continuously provided to a proximal end of the bending portion 12 and which is elongated along the longitudinal direction N.

Note that an observation lens, an illumination lens and the like, which are not shown, are provided in the distal end portion 11. Also, the bending portion 12 is bendable in two directions of up and down, for example, by rotation operation of a bending knob 14 provided to the operation section 3. Note that the bending portion 12 may alternatively be bendable in two directions of left and right.

Furthermore, a treatment instrument insertion opening 15 is provided to the operation section 3. The treatment instrument insertion opening 15 forms an opening at a proximal end of a treatment instrument insertion channel, not shown, inserted through the insertion section 2 and having an opening at a distal end surface of the distal end portion 11.

Accordingly, a treatment instrument that is inserted in the treatment instrument insertion channel through the treatment instrument insertion opening 15 protrudes into a subject from the opening at the distal end surface of the distal end portion 11.

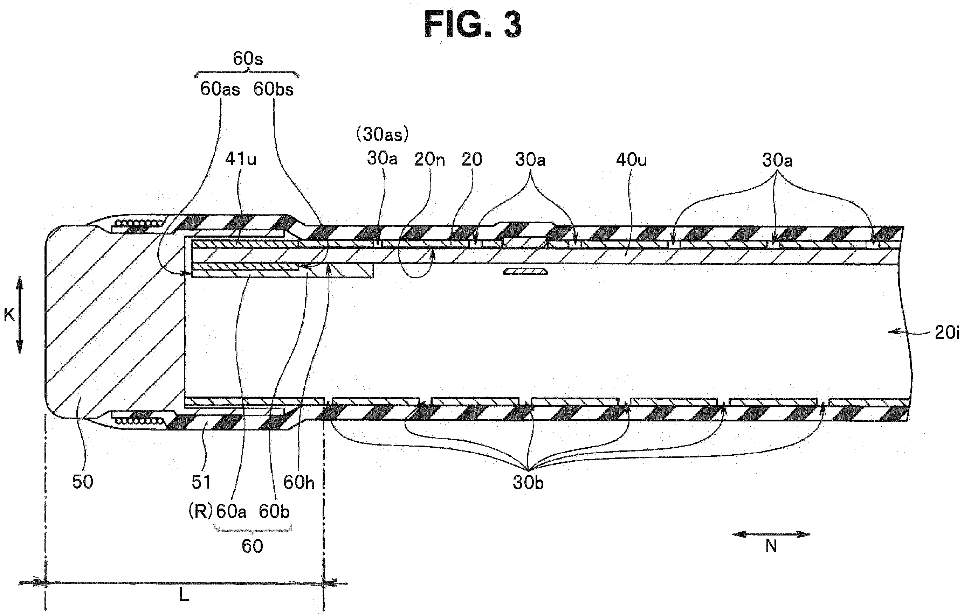

Note that, in addition to the treatment instrument insertion channel, known members which are normally provided in the insertion section of an endoscope, such as a light guide which is configured to transmit illumination light to the illumination lens described above, an image guide which is configured to transmit an optical image inside a subject focused on the observation lens described above to the eyepiece section 5, and pulling wires 40u, 40d (see Fig. 3; however, the pulling wire 40d is not shown) which are configured to bend the bending portion 12 and which are made of stainless steel, for example, are inserted through the insertion section 2 and the operation section 3. Note that the light guide described above is also inserted through the universal cord 4 and the connector 6.

Also, as shown in Fig. 2, the bending portion 12 includes a circular cylindrical member 20 which is a tubular bending member which is elongated along the longitudinal direction N, and which is formed of a super-elastic alloy, for example, to have a circular cylindrical shape.

Note that as the material forming the circular cylindrical member 20, nickel titanium (Ni-Ti), titanium alloy, β titanium, pure titanium, 64 titanium, A7075 and the like may be cited, but the material is not limited to the above as long as the material is a super-elastic alloy.

Furthermore, a plurality of slots 30 for bending (hereinafter simply referred to as "slot(s)") are formed at predetermined intervals in the longitudinal direction N by laser processing on the outer circumference of the circular cylindrical member 20. The slots 30 are formed as partial arcs along an outer circumferential direction C in a manner penetrating the outer circumference in a radial direction K of the circular cylindrical member 20 so as to communicate with an inner portion 20i of the circular cylindrical member 20.

More specifically, the slots 30 include a plurality of slots 30a which are formed, at predetermined intervals N1, N2 in the longitudinal direction N, as partial arcs of about 210 degrees, for example, in the outer circumferential direction C and on the UP side of the circular cylindrical member 20.

Also, the slots 30 include a plurality of slots 30b which are formed, at the predetermined intervals N1, N2 in the longitudinal direction N, as partial arcs of about 210 degrees, for example, in the outer circumferential direction C and on the DOWN side of the circular cylindrical member 20 while being shifted by about 180 degrees in the outer circumferential direction C and in a forward direction in the longitudinal direction N (hereinafter simply referred to as "forward") with respect to respective slots 30a.

Note that the slots 30a and the slots 30b are formed in such a way that the predetermined interval N2 in a back half region 20b of the circular cylindrical member 20, which is located rearward with respect to a front half region 20a in the longitudinal direction N (hereinafter simply referred to as "rearward"), is longer in the longitudinal direction N than the predetermined interval N1 in the front half region 20a (N2 > N1).

That is, the intervals of the slots 30a, 30b are smaller in the front half region 20a than in the back half region 20b, and thus, the bending radius of the circular cylindrical member 20 at the time of bending is smaller in the front half region 20a than in the back half region 20b.

As shown in Fig. 3 described below, the two pulling wires 40u, 40d described above are inserted through the circular cylindrical member 20, at positions shifted from each other in the outer circumferential direction C by about 180 degrees, that is, on the UP side and the DOWN side.

Also, at the distal end of each pulling wire 40u, 40d, a maximum outer diameter portion 41u, 41d (see Fig. 3; however, the maximum outer diameter portion 41d is not shown) provided at a distal end portion of each pulling wire 40u, 40d in the longitudinal direction N is engaged inside the distal end of the circular cylindrical member 20.

Note that the maximum outer diameter portion 41u, 41d is fixed, by swaging/crimping, to the distal end of the corresponding pulling wire 40u, 40d by covering the distal end portion of the corresponding pulling wire 40u, 40d with a pipe or the like.

Accordingly, for example, if the pulling wire 40u is pulled by the bending knob 14, because the predetermined interval N1 of the slots 30a in the front half region 20a is smaller than the predetermined interval N2 of the slots 30a in the back half region 20b, as described above, the bending portion 12 is bent to the UP side from the distal end side.

On the other hand, if the pulling wire 40d is pulled by the bending knob 14, because the predetermined interval N1 of the slots 30b in the front half region 20a is smaller than the predetermined interval N2 of the slots 30b in the back half region 20b, as described above, the bending portion 12 is bent to the DOWN side from the distal end side.

Next, an engaging structure at the distal end of the pulling wire 40u, 40d will be described with reference to Fig. 3. Fig. 3 is a diagram focusing on the circular cylindrical member, and schematically showing a part of a cross-section of the insertion section, taken along line III-III in Fig. 1. Note that, in Fig. 3, only the engaging structure at the distal end of the pulling wire 40u is shown as an example for the sake of simplicity in the drawing.

As shown in Fig. 3, the outer circumference of the distal end side of the circular cylindrical member 20 is fitted and fixed in an inner circumference of the proximal end side of a distal end rigid member 50, which is another tubular member constituting the distal end portion 11. Furthermore, an outer circumference of the distal end rigid member 50 and the outer circumference of the circular cylindrical member 20 are covered by an outer skin 51, a distal end of which is fixed by bobbin bonding or the like to the outer circumference of the distal end rigid member 50.

Furthermore, a protrusion portion 60 is provided, on the UP side of the outer circumference on the distal end side of the circular cylindrical member 20, protruding inward in the radial direction K with respect to an inner circumferential surface 20n of the circular cylindrical member 20. A wire hole 60h penetrating in the longitudinal direction N is provide to the protrusion portion 60.

The maximum outer diameter portion 41u of the pulling wire 40u inserted through the wire hole 60h is engaged with at least a part of a distal end surface 60s of the protrusion portion 60.

Note that the pulling wire 40u is assembled inside the insertion section 2 in such a manner that the maximum outer diameter portion 41u fixed at the distal end portion of the pulling wire 40u is engaged with the distal end surface 60s after the pulling wire 40u is inserted rearward from the forward side.

The protrusion portion 60 includes a wire engaging portion 60b which is engaged with the maximum outer diameter portion 41u at a distal end surface 60bs constituting the distal end surface 60s, and which includes a wire hole 60h.

Moreover, the protrusion portion 60 includes a guide member 60a which is integrally formed with the wire engaging portion 60b, which includes a distal end surface 60as constituting the distal end surface 60s, and which protrudes forward with respect to the wire engaging portion 60b.

The guide member 60a constitutes a restriction portion R which is located inward in the radial direction K with respect to the maximum outer diameter portion 41u inside the distal end of the circular cylindrical member 20, and which is configured to restrict inward movement, in the radial direction K, of the maximum outer diameter portion 41u engaged with the distal end surface 60bs of the wire engaging portion 60b at the time when the pulling wire 40u is pulled.

Note that an engaging structure at the distal end of the pulling wire 40d is the same as the engaging structure at the distal end of the pulling wire 40u described above.

That is, although not shown, a maximum outer diameter portion 41d fixed to the distal end of the pulling wire 40d is engaged with a distal end surface 60bs of a wire engaging portion 60b of a protrusion portion 60 which protrudes inward in the radial direction K from the DOWN side of the outer circumference on the distal end side of the circular cylindrical member 20 and to which a wire hole 60h through which the pulling wire 40d is to be inserted is formed along the longitudinal direction N. Furthermore, inward movement, in the radial direction K, of the maximum outer diameter portion 41d is restricted by a guide member 60a.

Note that other components are the same as the components of a general endoscope, and description of such components is omitted.

As described above, in the present embodiment, at the distal end of the pulling wire 40u, 40d, the maximum outer diameter portion 41u, 41d provided at the distal end portion of the corresponding pulling wire 40u, 40d is engaged with at least a part of the distal end surface 60s of the corresponding protrusion portion 60 protruding inward in the radial direction K on the distal end side of the circular cylindrical member 20.

Furthermore, inward movement, in the radial direction K, of the maximum outer diameter portion 41u, 41 d engaged with the corresponding distal end surface 60s is restricted by the guide member 60a of the corresponding protrusion portion 60.

Therefore, in the case where the material of the pulling wire 40u, 40d and the material of the circular cylindrical member 20 are different from each other, the distal end of the pulling wire 40u, 40d may be engaged inside the distal end of the circular cylindrical member 20 by the corresponding protrusion portion 60 without having to use, as in a conventional case, a coupling member, of the same material as the pulling wire 40u, 40d, provided between the distal end rigid member 50 and the circular cylindrical member 20 in the longitudinal direction N.

Accordingly, because a coupling member does not have to be used to fix the distal end of the pulling wire 40u, 40d, a distal end rigid length L (see Fig. 3) is not made longer than in a conventional case.

Furthermore, when a rearward pulling force is applied to the pulling wire 40u, 40d, the maximum outer diameter portion 41u, 41d engaged with the corresponding distal end surface 60bs is sometimes moved inward in the radial direction K.

However, in the present embodiment, inward movement of the maximum outer diameter portion 41u, 41d in the radial direction K may be prevented by the guide member 60a of the corresponding protrusion portion 60, and thus, the maximum outer diameter portion 41u, 41d may be prevented from interfering with the internal component of the circular cylindrical member 20.

The bending portion 12 having a configuration according to which the distal ends of the pulling wires 40u, 40d may be fixed to the circular cylindrical member 20 without affecting the distal end rigid length and according to which inward movement, in the radial direction, of the maximum outer diameter portions 41u, 41d of the pulling wires 40u, 40d may be prevented may thus be provided.

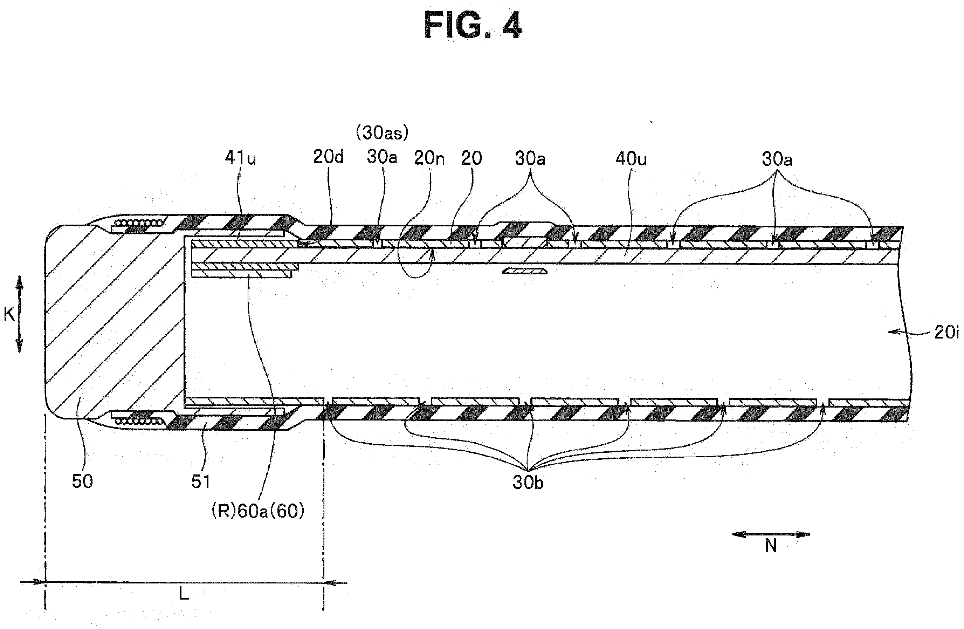

A modification will be described below with reference to Fig. 4. Fig. 4 is a partial cross-sectional view of the distal end side of the insertion section, showing a modification where the protrusion portion in Fig. 3 is formed only from the guide member. Note that, also in Fig. 4, only the engaging structure at the distal end of the pulling wire 40u is shown as an example for the sake of simplicity in the drawing.

In the present embodiment described above, the protrusion portion 60 is described to be configured with the guide member 60a and the wire engaging portion 60b. However, the protrusion portion 60 may alternatively be configured only by the guide member 60a.

More specifically, as shown in Fig. 4, the protrusion portion 60 may be configured only by the guide member 60a which is formed by pressing inward, by stamping or the like, in the radial direction K, a part of the UP side of the outer circumference on the distal end side of the circular cylindrical member 20.

In such a case, the maximum outer diameter portion 41u cannot be engaged with the distal end surface 60s of the protrusion portion 60 as in the present embodiment.

However, as shown in Fig. 4, the maximum outer diameter portion 41u may be engaged with an opening end portion 20d which is formed on the outer circumference of the circular cylindrical member 20 by pressing down the guide member 60a. Note that the configuration described above is also applied to the protrusion portion 60 which is provided on the DOWN side of the circular cylindrical member 20.

The configuration described above may achieve the same effect as the effect of the present embodiment described above, and also, the configuration may facilitate formation of the protrusion portion 60 because the guide member 60a can be formed by pressing inward, by stamping or the like, in the radial direction K, a part of the UP side of the outer circumference on the distal end side of the circular cylindrical member 20 and the wire engaging portion 60b does not have to be separately formed.

### (Second Embodiment)

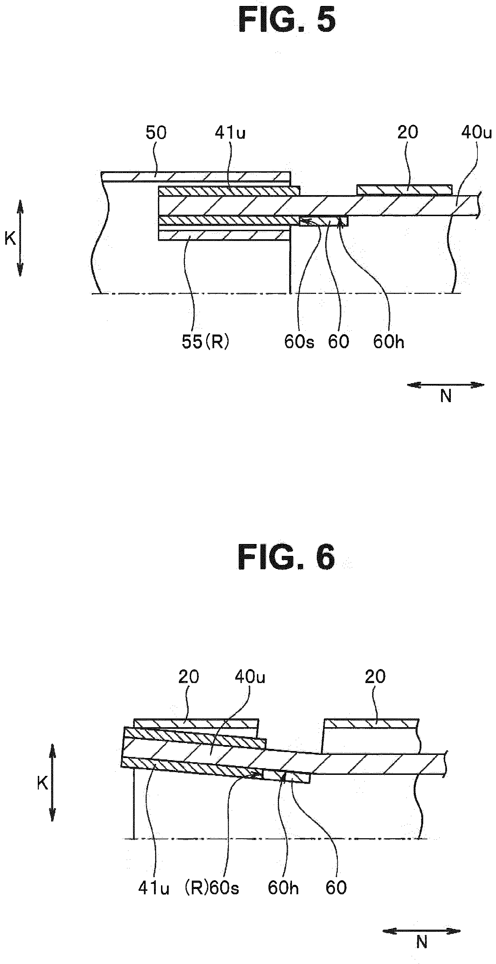

Fig. 5 is a partial cross-sectional view showing a circular cylindrical member constituting a bending portion of a present embodiment, together with a distal end rigid portion and a pulling wire, a maximum outer diameter portion of which is fixed to a distal end portion.

Compared to the bending portion of the first embodiment shown in Figs. 1 to 3 described above, the configuration of the bending portion of the second embodiment is different in that a guide member as a restriction portion is integrally formed not with a protrusion portion but with a distal end rigid member.

Description will therefore be given only on the difference, and components the same as the components of the first embodiment will be denoted by the same reference signs, and description of the components will be omitted. Note that, also in Fig. 5, only the engaging structure at the distal end of the pulling wire 40u is shown as an example for the sake of simplicity in the drawing.

As shown in Fig. 5, in the present embodiment, the protrusion portion 60 is configured only by a member to which the wire hole 60h is formed and the distal end surface 60s of which is to be engaged with the maximum outer diameter portion 41u. That is, the protrusion portion 60 is configured only by a member corresponding to the wire engaging portion 60b of the first embodiment described above.

Note that the protrusion portion 60 of the present embodiment is formed by pressing inward, by stamping or the like, in the radial direction K, a part of the UP side of the outer circumference on the distal end side of the circular cylindrical member 20.

Furthermore, a guide member 55 which is provided inside the circular cylindrical member 20, on the inner side in the radial direction K with respect to the maximum outer diameter portion 41u inside the proximal end side of the distal end rigid member 50, which is located on the inner side in the radial direction K with respect to the maximum outer diameter portion 41u, and which restricts inward movement, in the radial direction K, of the maximum outer diameter portion 41u engaged with the protrusion portion 60 is integrally formed with the distal end rigid member 50. Note that, in the present embodiment, the guide member 55 configures the restriction portion R.

Note that the same configuration as the configuration described above is applied to the protrusion portion 60 and the guide member 55 provided on the DOWN side of the circular cylindrical member 20. Also, other components are the same as the components of the first embodiment described above.

The configuration described above may achieve the same effect as the effect of the first embodiment described above, and also, the configuration may simplify the configuration of the circular cylindrical member 20 compared to the first embodiment described above because the guide member 55 may be provided to other than the protrusion portion 60 provided to the circular cylindrical member 20.

### (Third Embodiment)

Fig. 6 is a partial cross-sectional view showing a circular cylindrical member constituting a bending portion of a present embodiment, together with a pulling wire, a maximum outer diameter portion of which is fixed to a distal end portion.

Compared to the bending portion of the first embodiment shown in Figs. 1 to 3 and the bending portion of the second embodiment shown in Fig. 5, which are described above, the configuration of the bending portion of the third embodiment is different in that a restriction portion is formed at the distal end surface of a protrusion portion.

Description will therefore be given only on the difference, and components the same as the components of the first and the second embodiments will be denoted by the same reference signs, and description of the components will be omitted. Note that, also in Fig. 6, only the engaging structure at the distal end of the pulling wire 40u is shown as an example for the sake of simplicity in the drawing.

As shown in Fig. 6, in the present embodiment, the protrusion portion 60 is configured only by a member to which the wire hole 60h is formed and the distal end surface 60s of which is to be engaged with the maximum outer diameter portion 41u.

Furthermore, the protrusion portion 60 is formed by pressing inward, by stamping or the like, in the radial direction K, a member which includes the wire hole 60h, which is integrated with the UP side of the outer circumference of the circular cylindrical member 20, and which protrudes inward in the radial direction from the inner circumferential surface 20n.

Moreover, the distal end surface 60s, of the protrusion portion 60, where the maximum outer diameter portion 41u is to be engaged is formed to have an inclined shape, with the inner side, in the radial direction K, located forward in the longitudinal direction N with respect to the outer side.

Accordingly, when the maximum outer diameter portion 41u is engaged with the distal end surface 60s, the maximum outer diameter portion 41u is prevented, by the shape of the distal end surface 60s inclined outward in the radial direction K, from moving inward in the radial direction K.

That is, in the present embodiment, the distal end surface 60s configures the restriction portion R which is configured to restrict the inward movement, in the radial direction K, of the maximum outer diameter portion 41u which is engaged with the protrusion portion 60.

Note that the shape of the distal end surface 60s is not limited to a linearly inclined shape, and may alternatively be a stepwise shape having step(s) or the like as long as the inward movement, in the radial direction K, of the maximum outer diameter portion 41u which is engaged with the protrusion portion 60 may be restricted.

Furthermore, the inclined shape of the distal end surface 60s may be formed by post-processing the distal end surface 60s after performing stamping mentioned above. Alternatively, an inclined slit may be formed in advance at a position, on the UP side of the outer circumference on the distal end side of the circular cylindrical member 20, where stamping is to be performed, and by performing stamping along the inclined slit, the protrusion portion 60 may be integrally formed at the time of stamping.

Note that the same configuration as the configuration described above is applied to the protrusion portion 60 provided on the DOWN side of the circular cylindrical member 20. Also, other components are the same as the components of the first embodiment described above.

The configuration described above may achieve the same effect as the effects of the first and the second embodiments described above, and also, the structures of the circular cylindrical member 20 and the distal end rigid member 50 are simplified because the guide member becomes unnecessary due to the restriction portion R being formed at the distal end surface 60s.

### (Fourth Embodiment)

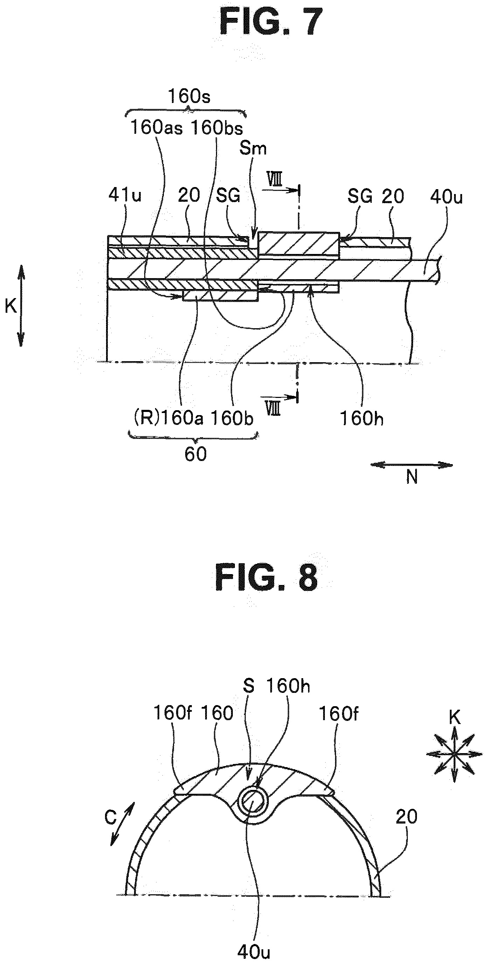

Fig. 7 is a partial cross-sectional view showing a circular cylindrical member constituting a bending portion of a present embodiment, together with a pulling wire, a maximum outer diameter portion of which is fixed to a distal end portion, and Fig. 8 is a cross-sectional view of the circular cylindrical member and a protrusion portion, taken along line VIII-VIII in Fig. 7.

Compared to the bending portion of the first embodiment shown in Figs. 1 to 3 described above, the configuration of the bending portion of the fourth embodiment is different in that a protrusion portion separate from a circular cylindrical member is inserted in the radial direction from outside the circular cylindrical member, through a groove of the circular cylindrical member.

Description will therefore be given only on the difference, and components the same as the components of the first embodiment will be denoted by the same reference signs, and description of the components will be omitted. Note that, also in Figs. 7 and 8, only the engaging structure at the distal end of the pulling wire 40u is shown as an example for the sake of simplicity in the drawings.

As shown in Figs. 7 and 8, a groove S penetrating the outer circumference in the radial direction K and having a rectangular shape in plan view, for example, is formed on the UP side of the outer circumference on the distal end side of the circular cylindrical member 20, and a protrusion portion 160 is inserted into the circular cylindrical member 20 through the groove S.

The protrusion portion 160 includes flange portions 160f which are engaged with outer circumferential edges SG of the groove S on the outer circumference of the circular cylindrical member 20 when the protrusion portion 160 is inserted into the circular cylindrical member 20 through the groove S, and the flange portions 160f are joined and fixed to the outer circumference of the circular cylindrical member 20, for example.

At this time, a wire hole 160h formed to the protrusion portion 160 in a manner penetrating the protrusion portion 160 along the longitudinal direction N is disposed inside the circular cylindrical member 20 according to engagement of the flange portions 160f with the outer circumferential edges SG.

Also, the maximum outer diameter portion 41u of the pulling wire 40u inserted through the wire hole 160h is engaged with at least a part of a distal end surface 160s of the protrusion portion 160.

The protrusion portion 160 includes a wire engaging portion 160b which is engaged with the maximum outer diameter portion 41u at a distal end surface 160bs constituting the distal end surface 160s, and which includes the wire hole 160h and the flange portions 160f.

Moreover, the protrusion portion 160 includes a guide member 160a which is integrally formed with the wire engaging portion 160b, which includes a distal end surface 160as constituting the distal end surface 160s, and which protrudes forward with respect to the wire engaging portion 160b.

The guide member 160a is located inward in the radial direction K with respect to the maximum outer diameter portion 41u inside the distal end of the circular cylindrical member 20, restricts inward movement, in the radial direction K, of the maximum outer diameter portion 41u engaged with the distal end surface 160bs of the wire engaging portion 160b, and constitutes the restriction portion R.

Furthermore, as shown in Fig. 7, the wire engaging portion 160b of the protrusion portion 160 is in close contact with the outer circumferential edge SG located rearward with respect to the circular cylindrical member 20, and thus, a gap Sm is formed between the wire engaging portion 160b and the outer circumferential edge SG located forward with respect to the circular cylindrical member 20.

Accordingly, also in the case of the protrusion portion 160d inserted through the groove S from outside the circular cylindrical member 20, the protrusion portion 160 is capable of holding the maximum outer diameter portion 41u when a great pulling force is applied to the pulling wire 40u, even though the protrusion portion 160 is not joined and fixed to the outer circumference of the circular cylindrical member 20.

Note that the same configuration as the configuration described above is applied to the protrusion portion 160 provided on the DOWN side of the circular cylindrical member 20. Also, other components are the same as the components of the first embodiment described above.

The configuration described above may achieve the same effect as the effect of the first embodiment described above, and also, the configuration may simplify the shape of the circular cylindrical member 20 because the protrusion portion 160 can be formed separately from the circular cylindrical member 20.

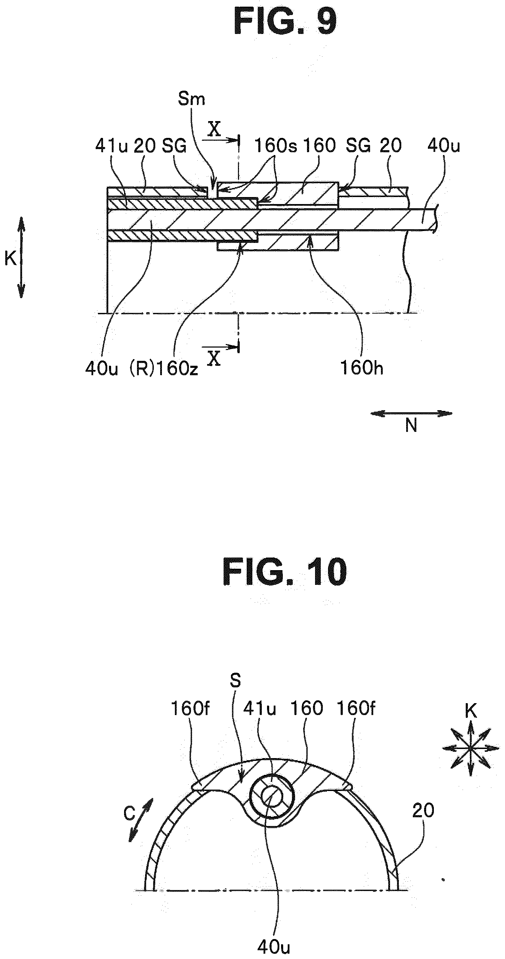

A modification will be described below with reference to Figs. 9 and 10. Fig. 9 is a partial cross-sectional view showing a modification of the configuration of the circular cylindrical member in FIg.8, together with a pulling wire, a maximum outer diameter portion of which is fixed to a distal end portion, and Fig. 10 is a cross-sectional view of the circular cylindrical member and a protrusion portion, taken along line X-X in Fig. 9. Note that, also in Figs. 9 and 10, only the engaging structure at the distal end of the pulling wire 40u is shown as an example for the sake of simplicity in the drawings.

In the present embodiment described above, the protrusion portion 160 is described to be configured from the wire engaging portion 160b and the guide member 160a.

However, as shown in Figs. 9 and 10, the protrusion portion 160 may alternatively be configured by one member which is engaged with the maximum outer diameter portion 41u at the distal end surface 160s, and on which the wire hole 160h is formed.

Note that a counterbore hole 160z is formed at a part, of the distal end surface 160s, where the maximum outer diameter portion 41u is to be engaged, and the maximum outer diameter portion 41u is engaged with a bottom surface of the counterbore hole 160z.

Accordingly, even though the guide member 160a is not provided to the protrusion portion 160, inward movement, in the radial direction K, of the maximum outer diameter portion 41u engaged with the distal end surface 160s is restricted by the counterbore hole 160z. According to the present configuration, the counterbore hole 160z thus configures the restriction portion R.

Note that the same configuration as the configuration described above is applied to the protrusion portion 160 provided on the DOWN side of the circular cylindrical member 20. Also, other components are the same as the components of the present embodiment described above.

The configuration described above may achieve the same effect as the effect of the present embodiment described above, and also, the shape of the protrusion portion 160 may be simplified because the guide member 160a does not have to be provided to the protrusion portion 160.

### (Fifth Embodiment)

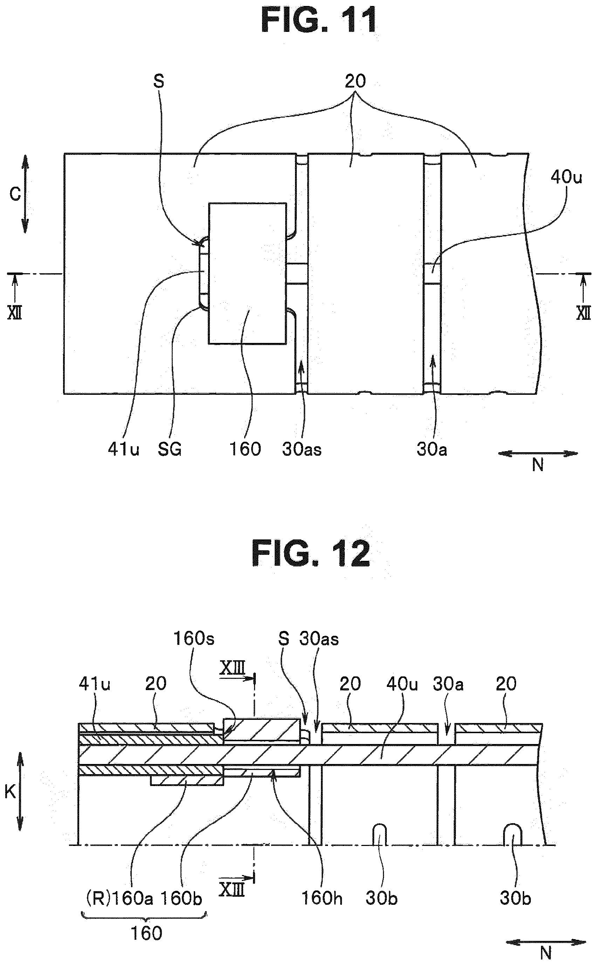

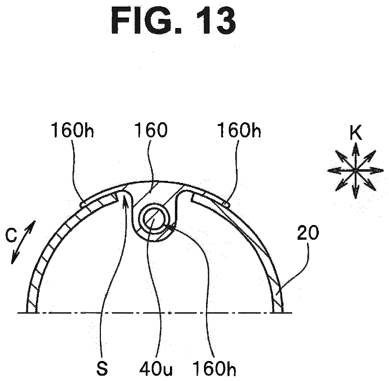

Fig. 11 is a plan view schematically showing, from an UP side, an external appearance of a distal end side of a circular cylindrical member constituting a bending portion of a present embodiment, Fig. 12 is a diagram showing a cross-section of the circular cylindrical member, taken along line XII-XII in Fig. 11, together with a pulling wire, a maximum outer diameter portion of which is fixed to a distal end portion, and Fig. 13 is a cross-sectional view of the circular cylindrical member and a protrusion portion, taken along line XIII-XIII in Fig. 12.

Compared to the bending portion of the fourth embodiment shown in Figs. 7 and 8 described above, the configuration of the bending portion of the fifth embodiment is different in that a groove, formed on the circular cylindrical member, in which the protrusion portion is to be inserted communicates with a first slot at the most distal end.

Description will therefore be given only on the difference, and components the same as the components of the fourth embodiment will be denoted by the same reference signs, and description of the components will be omitted. Note that, also in Figs. 11 to 13, only the engaging structure at the distal end of the pulling wire 40u is shown as an example for the sake of simplicity in the drawings.

As shown in Figs. 11 and 12, in the present embodiment, a groove S communicates with a first slot 30as at the most distal end, among a plurality of slots 30a.

Also, as shown in Figs. 11 to 13, the protrusion portion 160 inserted in the circular cylindrical member 20 through the groove S is joined and fixed to the outer circumference of the circular cylindrical member 20 in a state where the flange portions 160f are engaged with the outer circumferential edges SG of the groove S.

Note that, because the groove S communicates with the first slot 30as, the protrusion portion 160 is not engaged with the rearward outer circumferential edge SG of the groove S as in the fourth embodiment described above.

Because the protrusion portion 160 resists a great rearward pulling force that is applied through the pulling wire 40u of the maximum outer diameter portion 41u engaged with the distal end surface 160s, the flange portions 160f are joined and fixed to the outer circumference of the circular cylindrical member 20 with a great contact area.

Note that the same configuration as the configuration described above is applied to the protrusion portion 160 provided on the DOWN side of the circular cylindrical member 20. That is, a groove S formed on the DOWN side communicates with a first slot at the most distal end, among a plurality of slots 30b. Also, other components are the same as the components of the fourth embodiment described above.

In the fourth embodiment described above, such a configuration results in a disadvantage that the further separated the groove S is from the first slot 30as in the longitudinal direction N, the more the distal end rigid length is increased, so as to secure strength of a part between the groove S on the outer circumference of the circular cylindrical member 20 and the first slot 30as.

In contrast, with the configuration of the present embodiment, a thin portion is not formed on the outer circumference of the circular cylindrical member 20 between the groove S and the first slot 30as, and thus, the groove S allowing the protrusion portion 160 to be inserted into the circular cylindrical member 20 may be formed on the outer circumference of the circular cylindrical member 20 without increasing the distal end rigid length. Note that the components are the same as the components of the fourth embodiment described above.

### (Sixth Embodiment)

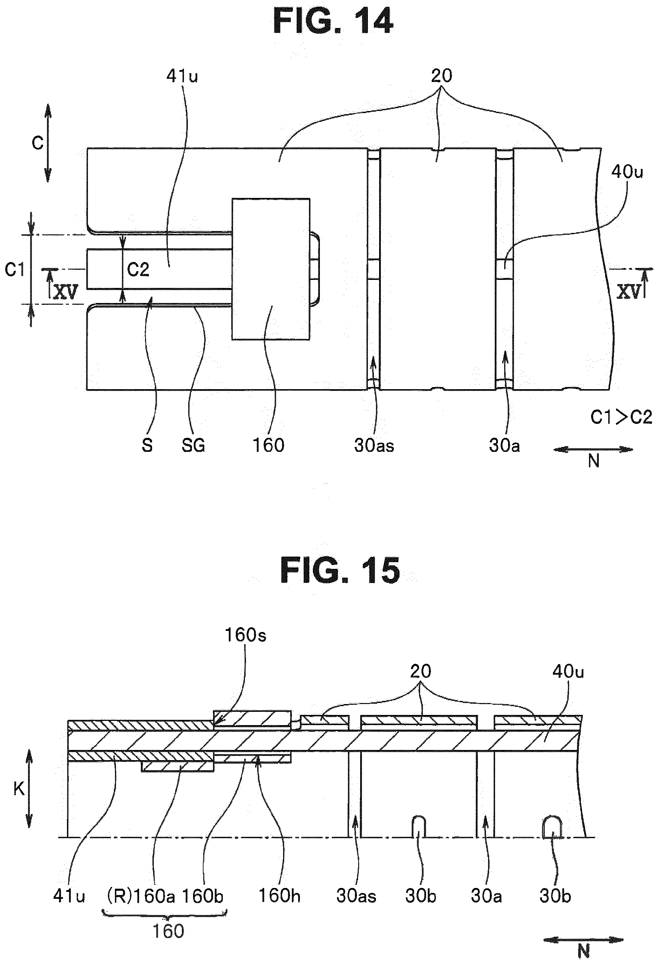

Fig. 14 is a plan view schematically showing, from an UP side, an external appearance of a distal end side of a circular cylindrical member constituting a bending portion of a present embodiment, and Fig. 15 is a diagram showing a cross-section of the circular cylindrical member, taken along line XV-XV in Fig. 14, together with a pulling wire, a maximum outer diameter portion of which is fixed to a distal end portion.

Compared to the bending portion of the fifth embodiment shown in Figs. 11 to 13 described above, the configuration of the bending portion of the sixth embodiment is different in that the maximum outer diameter portion of the pulling wire is caused to fall into a groove of the circular cylindrical member.

Description will therefore be given only on the difference, and components the same as the components of the fifth embodiment will be denoted by the same reference signs, and description of the components will be omitted. Note that, also in Figs. 14 and 15, only the engaging structure at the distal end of the pulling wire 40u is shown as an example for the sake of simplicity in the drawings.

As shown in Figs. 14 and 15, in the present embodiment, the groove S is formed up to the distal end of the circular cylindrical member 20, and a width C1 in the outer circumferential direction C is formed to be greater than a diameter C2 of the maximum outer diameter portion 41u (C1 > C2).

The maximum outer diameter portion 41u thereby falls entirely into the groove S when the maximum outer diameter portion 41u is engaged with the distal end surface 60s of the protrusion portion 60.

Note that, to prevent the maximum outer diameter portion 41u from greatly projecting outward in the radial direction K through the groove S, the outer circumference of the circular cylindrical member 20 on which the groove S is formed has to be covered by another tubular member, for example.

Note that the same configuration as the configuration described above is applied to the groove S provided on the DOWN side of the circular cylindrical member 20. Also, other components are the same as the components of the fifth embodiment described above.

According to such a configuration, because the entire maximum outer diameter portion 41u falls into the groove S, inward movement of the maximum outer diameter portion 41u in the radial direction K may be more reliably prevented even if the guide member 160a is not provided.

Moreover, because the maximum outer diameter portion 41u falls into the groove S, a larger space may be secured inside the circular cylindrical member 20 compared to the fifth embodiment. Note that the effects are the same as the effects of the fifth embodiment described above.

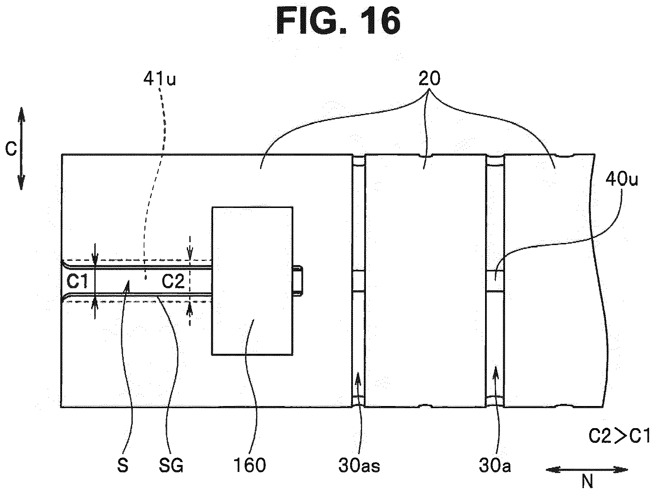

A modification will be described below with reference to Fig. 16. Fig. 16 is a plan view schematically showing, from an UP side, an external appearance of a distal end side of a circular cylindrical member of a modification according to which a groove width in Fig. 15 is made smaller than a maximum outer diameter portion.

As shown in Fig. 16, as completely opposed to the present embodiment, the diameter C2 of the maximum outer diameter portion 41u may be formed to be greater than the width C1 of the groove S in the outer circumferential direction C (C2 > C1).

Note that the same configuration as the configuration described above is applied to the groove S provided on the DOWN side of the circular cylindrical member 20. Also, other components are the same as the components of the present embodiment described above.

The same effect as the effect of the present embodiment described above may thus be achieved, and also, because only a part of the maximum outer diameter portion 41u is caused to fall into the groove S, the space inside the circular cylindrical member 20 is reduced compared to the present embodiment but the maximum outer diameter portion 41u may be prevented from projecting outward, through the groove S, in the radial direction K even though another tubular member is not used as a cover as opposed to the present embodiment.

Note that, in the first to the sixth embodiments described above, the circular cylindrical member 20 is cited as an example of the tubular member for bending, but the tubular member for bending is not limited only to the example described above such an example is not restrictive, and the present embodiments are applicable also in a case where the tubular member for bending is configured by a plurality of bending pieces of a material different from the material of the pulling wire, and where the distal end of the pulling wire cannot be welded to the bending piece.

Furthermore, an example is cited where the bending portion 12 is bent in two directions, but application is, of course, possible to a bending portion which is bent in four directions.

The present application claims priority from Japanese Patent Application No. 2015-109049 filed in Japan on May 28, 2015, the entire contents of which are incorporated in the present specification, claims, and drawings by reference.

## Claims

1. An endoscope bending portion comprising:
a tubular member for bending;
a protrusion portion that is formed protruding on an inner circumference of the tubular member for bending, on a distal end side in a longitudinal direction, and that has a wire hole formed in the longitudinal direction;
a pulling wire that has a maximum outer diameter portion formed at a distal end portion in the longitudinal direction, and that is inserted through the wire hole to have the maximum outer diameter portion engaged with at least a part of a distal end surface of the protrusion portion; and
a restriction portion that is provided inside the tubular member for bending, and that restricts inward movement in the tubular member for bending, in a radial direction, of the maximum outer diameter portion engaged with the protrusion portion.

2. The endoscope bending portion according to claim 1, wherein
the restriction portion is formed on the distal end surface of the protrusion portion, and
the distal end surface is formed to have an inclined shape, with an inner side of the distal end surface, in the radial direction, located forward in the longitudinal direction with respect to an outer side.

3. The endoscope bending portion according to claim 1, wherein the restriction portion is a guide member located on an inner side in the radial direction with respect to the maximum outer diameter portion.

4. The endoscope bending portion according to claim 3, wherein the guide member is integrally formed with the protrusion portion.

5. The endoscope bending portion according to claim 3, wherein
another tubular member is fitted on the distal end side of the tubular member for bending, and
the guide member is integrally formed with the other tubular member.

6. The endoscope bending portion according to claim 1, wherein
a groove is formed on an outer circumference on the distal end side of the tubular member for bending in a manner penetrating the outer circumference in the radial direction, and the protrusion portion includes a flange portion that is engaged with an outer circumferential edge of the groove at the outer circumference of the tubular member for bending in a state where the protrusion portion is inserted through the groove into the tubular member for bending from outside in the radial direction, and
the wire hole is disposed inside the tubular member for bending according to engagement of the flange portion with the outer circumferential edge of the groove.

7. The endoscope bending portion according to claim 6, wherein
a plurality of slots for bending are formed, in a manner penetrating in the radial direction, in the tubular member for bending, at predetermined intervals in the longitudinal direction, and
the groove communicates with a first slot at a most distal end in the longitudinal direction, among the plurality of slots for bending.

8. The endoscope bending portion according to claim 7, wherein at least a part of the maximum outer diameter portion is caused to fall into the groove.

9. The endoscope bending portion according to claim 8, wherein a diameter of the maximum outer diameter portion is formed to be greater than a width of the groove in an outer circumferential direction of the tubular member for bending.

10. The endoscope bending portion according to any one of claims 1 to 9, wherein the tubular member for bending is made of a super-elastic alloy.
